# EUROPÄISCHE PATENTSCHRIFT

(11) **EP 0 710 644 B1**
(45) Veröffentlichungstag und Bekanntmachung des Hinweises auf die Patenterteilung: **24.09.1997**
(21) Anmeldenummer: 95116655.2
(22) Anmeldetag: 23.10.1995
(51) Int. Cl.: C07C 213/02, C07C 215/80, C07C 205/12, C07C 205/37

(54) **Verfahren zur Herstellung von 4,6-Diaminoresorcin**
Process for the preparation of 4,6-diaminoresorcinol
Procédé pour la préparation de 4,6-diaminorésorcinol

(30) Priorität: 03.11.1994 DE 4439185; 03.11.1994 DE 4439194; 03.11.1994 DE 4439191
(43) Veröffentlichungstag der Anmeldung: 08.05.1996
(73) Patentinhaber: BAYER AG, 51368 Leverkusen (DE)
(72) Erfinder: Behre, Horst, Dr., D-51519 Odenthal (DE); Fiege, Helmut, Dr., D-51373 Leverkusen (DE); Blank, Heinz-Ulrich, Dr., D-51519 Odenthal (DE); Heinz, Uwe, Dr., D-51061 Köln (DE); Eymann, Wolfgang, Dr., D-51061 Köln (DE)

(56) Entgegenhaltungen:
- EP-A- 0 312 931
- EP-A- 0 402 688
- US-A- 5 414 130
- US-H- H 726
- PATENT ABSTRACTS OF JAPAN vol. 950 no. 2 & JP-A-07 048321 (NISSAN CHEM IND LTD) 21.Februar 1995,
- 'Methoden der organischen Chemie (Houben-weyl), Band IV/1c ' 1980 , EUGEN MÜLLER ET AL. , GEORG THIEME VERLAG STUTTGART . NEW YORK * Seite 509 *

## Beschreibung

Die vorliegende Erfindung betrifft ein Verfahren zur Herstellung von 4,6-Diaminoresorcin aus 1,3-Dichlorbenzol durch Dinitrierung zu einem 1,3-Dichlor-4,6/2,4-dinitrobenzol-Gemisch, anschließende Umsetzung mit Natrium-benzylat zum 1,3-Dibenzyloxy-4,6-dinitrobenzol und katalytische Hydrierung.

4,6-Diaminoresorcin ist ein wichtiger Monomerbaustein für Kunststoffe, insbesondere Polybenzoxazole (Macromolecules 1981, 915).

In EP 0 402 688 ist die Synthese von 4,6-Diaminoresorcin aus 1,3-Dichlor-4,6-dinitrobenzol durch Umsetzung mit Natrium-benzylat zum 1,3-Dibenzyloxy-4,6-dinitrobenzol und anschließende katalytische Hydrierung beschrieben. Nachteilig an dem beschriebenen Verfahren ist, daß für die Synthese reines 1,3-Dichlor-4,6-dinitrobenzol erforderlich ist, das nach Dinitrierung von 1,3-Dichlorbenzol zum 4,6/2,4-Dinitro-Isomerengemisch nur durch Ausbeute-mindernde Umlösung z.B. aus Ethanol erhalten werden kann. Eine Nacharbeitung der Beispiele 1 und 2 aus EP 0 402 688 hat zudem ergeben, daß die Umsetzung von reinem 1,3-Dichlor-4,6-dinitrobenzol mit Na-Benzylat in Benzylalkohol bei 50 bzw. 55°C in einer unbekannten Nebenreaktion zu erheblicher Bildung von Benzaldehyd führt, was sich Ausbeute- und Qualitäts-mindernd auf die Bildung von 1,3-Dibenzyloxy-4,6-dinitrobenzol auswirkt. Ein weiterer Nachteil ist die beschriebene Ausführungsform der heterogenen katalytischen Hydrierung von 1,3-Dibenzyloxy-4,6-dinitrobenzol am Pd/A-Kohle-Kontakt als Ansatz-Hydrierung mit anschließender wässriger, salzsaurer Aufarbeitung der Hydrier-Ansätze, die keine wirtschaftliche Rückführung des teuren Edelmetall-Kontaktes zuläßt.

Es wurde nun gefunden, daß man 4,6-Diaminoresorcin aus 1,3-Dichlor-4,6-dinitrobenzol durch Umsetzung mit Na-Benzylat in Benzylalkohol zum 1,3-Dibenzyloxy-4,6-dinitrobenzol und katalytische Hydrierung von 1,3-Dibenzyloxy-4,6-dinitrobenzol am Edelmetall-Kontakt herstellen kann, wenn man reines 1,3-Dibenzyloxy-4,6-dinitrobenzol direkt aus 1,3-Dichlor-4,6/2,4-dinitrobenzol-Isomerengemischen, wie sie beispielsweise durch Dinitrierung von 1,3-Dichlorbenzol erhalten werden, durch schrittweise, Temperatur-gestufte Substitution der Chlor-Atome durch die Benzyloxy-Gruppe unter milden Bedingungen unter weitgehender Vermeidung der unerwünschten Nebenreaktion zum Benzaldehyd gewinnt und das 1,3-Dibenzyloxy-4,6-dinitrobenzol am Edelmetall-Kontakt in einem organischen Lösemittel bei erhöhtem Druck und erhöhter Temperatur durch eine katalytische Pumphydrierung zum 4,6-Diaminoresorcin umsetzt.

Die Erfindung betrifft ein Verfahren zur Herstellung von 4,6-Diaminoresorcin aus 1,3-Dichlorbenzol durch Dinitrierung zum 1,3-Dichlor-4,6-dinitrobenzol, durch nachfolgende Umsetzung mit Na-Benzylat in Benzylalkohol zum 1,3-Dibenzyloxy-4,6-dinitrobenzol und durch nachfolgende katalytische Hydrierung von 1,3-Dibenzyloxy-4,6-dinitrobenzol an einem Edelmetall-Kontakt, das dadurch gekennzeichnet ist, daß man
a) in einem ersten Schritt 1,3-Dichlorbenzol in wasserfreier Schwefelsäure, die 0 bis 10 Gew.-% freies SO₃ enthält, mit einer Salpetersäure, Schwefelsäure und 0,7 bis 1,5 Mol, bevorzugt 0,8 bis 1,2 Mol, besonders bevorzugt 0,9 bis 1,1 Mol SO₃ pro Mol Salpetersäure enthaltenden Mischsäure in einem Temperaturbereich von 0 bis 40°C umsetzt, wobei das Molverhältnis HNO₃ zu 1,3-Dichlorbenzol 2 bis 3 beträgt,
b) in einem zweiten Schritt die in a) erhaltenen 1,3-Dichlor-4,6/2,4-dinitrobenzol-Isomerengemische, die 0,1 bis 60 % 1,3-Dichlor-2,4-dinitrobenzol, bezogen auf das Gesamtgewicht des Isomerengemisches enthalten, mit 2 bis 5 Mol Benzylalkohol und 2 bis 5 Äquivalenten einer starken Base in Gegenwart eines inerten Lösungsmittels umsetzt, wobei man hierzu stufenweise zunächst im Temperaturbereich vom -15 bis +15°C und dann im Temperaturbereich von 20-40°C arbeitet und
c) in einem dritten Schritt das in b) erhaltene 1,3-Dibenzyloxy-4,6-dinitrobenzol an einem Edelmetall-Kontakt in einem inerten organischen Lösungsmittel bei einem H₂-Druck von 1 bis 100 bar und einer Temperatur von 20 bis 100°C durch eine katalytische Pumphydrierung zum 4,6-Diaminoresorcin umsetzt.

Wesentlich für die Durchführung des erfindungsgemäßen Verfahrens in Schritt a) ist, daß man die Nitrierung in wasserfreier Schwefelsäure durchführt, beispielsweise in 100 %iger Schwefelsäure oder in Schwefelsäure, die 0 bis 10 Gew.-% freies SO₃ enthält. Wesentlich ist ferner, daß man für die Nitrierung eine Mischsäure verwendet, die Salpetersäure, Schwefelsäure und mindestens 0,7 Mol SO₃ pro Mol Salpetersäure enthält. Vorzugsweise enthält die Mischsäure 0,8 bis 1,2 Mol SO₃ pro Mol Salpetersäure, besonders bevorzugt 0,9 bis 1,1 Mol SO₃. Solche Mischsäuren können durch Vermischung von Salpetersäure und Oleum hergestellt werden. Der Anteil Schwefelsäure in der Mischsäure kann beliebig gewählt werden; er beträgt im allgemeinen das 3- bis 10-fache des 1,3-Dichlorbenzols (Gew./Gew.).

Pro Mol 1,3-Dichlorbenzol werden mindestens 2,0 Mol Salpetersäure, beispielsweise 2 bis 3 Mol Salpetersäure, vorzugsweise 2,1 bis 2,5 Mol Salpetersäure, besonders bevorzugt 2,15 bis 2,30 Mol Salpetersäure, in Form der Mischsäure eingesetzt.

Die Durchführung von Schritt a) des erfindungsgemäßen Verfahrens kann in zwei verschiedenen Varianten erfolgen.

### Variante 1:

In eine Vorlage aus wasserfreier Schwefelsäure oder Schwefelsäure, die 0 bis 10 Gew.-% freies SO₃ enthält, wird zunächst 1,3-Dichlorbenzol eingetropft und anschließend die Mischsäure, welche Salpetersäure, Schwefelsäure und mindestens 0,7 Mol SO₃ pro Mol Salpetersäure enthält, eindosiert. Die Dosierzeiten für 1,3-Dichlorbenzol betragen 5 Minuten bis 5 Stunden, für die Mischsäure 15 Minuten bis 10 Stunden und hängen im wesentlichen von der Reaktionstemperatur und der Wirksamkeit des verwendeten Kühlaggregates sowie der Ansatzgröße ab. Bevorzugte Dosierzeiten für 1,3-Dichlorbenzol sind 15 bis 60 Minuten, für die Mischsäure 1 bis 3 Stunden.

### Variante 2:

In eine Vorlage aus wasserfreier Schwefelsäure oder Schwefelsäure, die 0 bis 10 Gew.-% freies SO₃ enthält, werden simultan 1,3-Dichlorbenzol und die Mischsäure, welche Salpetersäure, Schwefelsäure und mindestens 0,7 Mol SO₃ pro Mol Salpetersäure enthält, eindosiert. In dieser bevorzugten Variante beträgt die Simultan-Dosierzeit 15 Minuten bis 10 Stunden, bevorzugt 30 Minuten bis 3 Stunden, und hängt von der Reaktionstemperatur und der Wirksamkeit des verwendeten Kühlaggregates sowie der Ansatzgröße ab. Bei dieser bevorzugten Variante wird insbesondere bei der Vorlage von Schwefelsäure, die 0 bis 10 Gew.-% freies SO₃ enthält, die Bildung von 1,3-Dichlorbenzol-sulfonsäuren praktisch vollständig vermieden.

Die Temperatur von Schritt a) des erfindungsgemäßen Verfahrens liegt im Bereich von 0 bis 40°C, bevorzugt 10 bis 30°C, insbesondere 15 bis 25°C.

Die nach dem erfindungsgemäßen Verfahren erhaltenen Nitriergemische werden in an sich bekannter Form aufgearbeitet, beispielsweise durch Austragen auf Wasser oder ein Eis/Wasser-Gemisch, Filtration und Wasser-Wäsche.

Wesentlich für die Durchführung des erfindungsgemäßen Verfahrens in Schritt b) ist, daß man technische, in Schritt a) erhaltene 1,3-Dichlor-4,6/2,4-dinitrobenzol-Isomerengemische mit mindestens 2 Mol Benzylalkohol und mindestens 2 Äquivalenten einer starken Base gegebenenfalls in Gegenwart eines inerten Lösungsmittels umsetzt. Diese Isomerengemische enthalten im allgemeinen 0,1 bis 60 % 1,3-Dichlor-2,4-dinitrobenzol, bezogen auf das Gesamtgewicht des Gemisches. Bevorzugt werden Gemische mit 5 bis 25 % 1,3-Dichlor-2,4-dinitrobenzol eingesetzt. Ganz besonders bevorzugt ist ein Gemisch aus 8 bis 15 % 1,3-Dichlor-2,4-dinitrobenzol und 85 bis 92 % 1,3-Dichlor-4,6-dinitrobenzol.

Das 1,3-Dichlor-4,6/2,4-dinitrobenzol-Isomerengemisch wird mit 2 bis 5 Mol, bevorzugt mit 2 bis 3 Mol Benzylalkohol und mit 2 bis 5 Äquivalenten einer starken Base umgesetzt. Als starke Basen werden eine oder mehrere aus der Gruppe der Alkalimetalle, wie Lithium, Natrium, Kalium, Rubidium oder Cäsium, bevorzugt Natrium oder Kalium, der Erdalkalimetalle, wie Magnesium, Calcium, Strontium oder Barium, bevorzugt Magnesium oder Calcium, der Alkalimetallhydroxide, wie Lithiumhydroxid, Natriumhydroxid, Kaliumhydroxid, Rubidiumhydroxid, Cäsiumhydroxid, bevorzugt Natriumhydroxid oder Kaliumhydroxid, der Erdalkalimetallhydroxide, wie Magnesiumhydroxid, Calciumhydroxid, Strontiumhydroxid oder Bariumhydroxid, bevorzugt Magnesiumhydroxid oder Calciumhydroxid, der Alkalimetallcarbonate, wie Lithiumcarbonat, Natriumcarbonat, Kaliumcarbonat, Rubidiumcarbonat oder Cäsiumcarbonat, bevorzugt Natriumcarbonat oder Kaliumcarbonat, und der Erdalikalimetallcarbonate, wie Magnesiumcarbonat, Calciumcarbonat, Strontiumcarbonat oder Bariumcarbonat, bevorzugt Magnesiumcarbonat oder Calciumcarbonat, eingesetzt. In bevorzugter Weise wird eine starke Base aus der Gruppe der Alkalimetalle, der Alkalimetallhydroxide und Alkalimetallcarbonate, in besonders bevorzugter Weise aus der Gruppe Natriummetall, Natriumhydroxid und Natriumcarbonat, eingesetzt. Ebenso kann der Benzylalkohol auch direkt in Form seines Alkoholates eingesetzt werden.

Die Temperatur-gestufte Reaktion wird in der ersten Stufe im Temperaturbereich von -15 bis +15°C, bevorzugt 0 bis 15°C, insbesondere 5 bis 15°C, und in der zweiten Reaktionsstufe in einem Temperaturbereich von 20 bis 40°C, bevorzugt 25 bis 35°C durchgeführt.

Als inertes Lösungsmittel kommt ein aliphatischer, aromatischer oder alkylaromatischer Kohlenwasserstoff, ein halogenierter aromatischer Kohlenwasserstoff oder zusätzlicher Benzylalkohol über die genannten 2 bis 5 Mol hinaus in Frage. Beispiele für solche Lösungsmittel sind: Pentan, Hexan, Heptan, Octan, Decan, Dodecan und höhere geradkettige oder verzweigte aliphatische Kohlenwasserstoffe, sowie Gemische daraus, wie Ligroin oder Petrolether, Benzol, Toluol, Ethylbenzol, Chlorbenzol, Brombenzol, Dichlorbenzol, Dibrombenzol, Chlortoluol, Dichlortoluol oder Cyclohexan. Selbstverständlich können auch Gemische mehrerer der genannten Lösungsmittel eingesetzt werden.

In einer bevorzugten Ausführungsform kommt Benzylalkohol gleichzeitig als Reaktionspartner und Lösungsmittel zum Einsatz. Hierbei wird Benzylalkohol in einer Menge von 3 bis 20 Gew.-Teilen, bevorzugt 5 bis 15 Gew.-Teilen, bezogen auf 1 Gew.-Teil Dichlor-dinitrobenzol, eingesetzt. Selbstverständlich kann der Benzylalkohol auch im Gemisch mit den genannten inerten Lösungsmitteln verwendet werden.

Die Reaktion ist unabhängig vom Reaktionsdruck und wird daher zweckmäßig bei Normaldruck durchgeführt. Ein erhöhter Druck könnte dann sinnvoll sein, wenn ein niedrig siedendes Lösungsmittel verwendet wird. In diesem Falle wird bevorzugt unter dem sich automatisch einstellenden Eigendruck gearbeitet. Ein verminderter Druck ist dann zweckmäßig, wenn die Temperatur-gestufte Reaktion in der ersten und/oder zweiten Stufe bei einer konstanten Siedetemperatur unter Rückfluß durchgeführt werden soll.

Die erfindungsgemäße Temperatur-gestufte Reaktion von Dichlor-dinitrobenzol mit Benzylalkohol in Gegenwart der genannten Basen erfordert in der ersten Stufe bei einer Reaktionstemperatur von 5 bis 15°C im allgemeinen Reaktionszeiten von 15 Minuten bis 3 Stunden, in der zweiten Stufe bei einer Reaktionstemperatur von 25 bis 35°C im allgemeinen Reaktionszeiten von 30 Minuten bis 6 Stunden und ergibt hohe Ausbeuten, die 90 % überschreiten.

Das Reaktionsprodukt ist im Reaktionsmedium schwerlöslich und kann daher durch ein einfaches Filtrieren isoliert werden. Anschließend können die anorganischen Nebenprodukte (beispielsweise Alkali- und/oder Erdalkalichloride) durch Anschlämmen in Wasser und erneute Filtration entfernt werden.

Wesentlich für die Durchführung des erfndungsgemäßen Verfahrens in Schritt c) ist, daß man das 1,3-Dibenzyloxy-4,6-dinitrobenzol durch eine katalytische Pumphydrierung am Edelmetall-Kontakt in einem organischen Lösungsmittel bei erhöhtem Druck und erhöhter Temperatur zum 4,6-Diaminoresorcin umsetzt. Bei dieser Ausführungsform wird eine Lösung oder Suspension von 1,3-Dibenzyloxy-4,6-dinitrobenzol in dem organischen Lösungsmittel unter Hydrierbedingungen in einen Hydrier-Autoklaven eingepumpt, in dem eine Suspension des Edelmetall-Kontaktes im organischen Lösungsmittel oder einer Lösung von 4.6-Diaminoresorcin im organischen Lösungsmittel unter H₂-Druck vorgelegt ist.

Bei der katalytischen Hydrierung von 1,3-Dibenzyloxy-4,6-dinitrobenzol zu 4,6-Diaminoresorcin erfolgt eine gleichzeitige Reduktion der Nitrogruppen zu den Aminogruppen und eine Abspaltung der Benzylgruppen unter Bildung von Hydroxyl-Gruppen und Toluol. Als Katalysatoren werden Platinmetalle, wie Palladium, Platin, Rhodium oder Ruthenium, bevorzugt Palladium oder Platin, auf einem geeigneten Träger, eingesetzt. Geeignete Träger sind A-Kohle, SiO₂, Al₂O₃ und andere. Ganz besonders bevorzugt wird ein Palladium/A-Kohle-Kontakt eingesetzt. Der Platinmetall/Träger-Kontakt wird in einer Menge von 0,1 bis 10 Gew.-% des Platinmetalls, bevorzugt 0,2 bis 2 Gew.-%, bezogen auf das Gewicht von 1,3-Dibenzyloxy-4,6-dinitrobenzol, eingesetzt. Die Hydrierung wird bei einem H₂-Druck von 1 bis 100 bar, bevorzugt 5 bis 50 bar, insbesondere 10 bis 30 bar und im Temperaturbereich von 20 bis 100°C, bevorzugt 40 bis 80°C, durchgeführt.

Als flüssiges Reaktionsmedium für die Hydrierung kann ein Alkohol, ein Ether, ein aromatischer oder alkylaromatischer Kohlenwasserstoff, eine organische Säure oder ein Gemisch mehrerer von ihnen eingesetzt werden. Beispiele für solche flüssigen Reaktionsmedien sind Methanol, Ethanol, Propanol, Isopropylalkohol, Butanol, Isobutanol, Tetrahydrofuran, Dioxan, Benzol, Toluol, Xylol, Dimethoxyethan, Diethoxy-ethan, 1,2-Dimethoxypropan und andere Glykolmono- und -diether, Essigsäure, Propionsäure oder ein Gemisch mehrerer von ihnen. Im Falle wasserlöslicher flüssiger Reaktionsmedien kann der organische Anteil dieses Reaktionsmediums bis zu 75 Gew.-% durch Wasser ersetzt werden. Wesentlich ist jedoch, daß das flüssige Reaktionsmedium bei der Hydriertemperatur ein ausreichendes Lösevermögen für das gebildete 4,6-Diaminoresorcin besitzt, um eine einfache Abtrennung und Rückführung des Platinmetall/Träger-Kontaktes beispielsweise durch Abdrücken und Filtration des in Lösung befindlichen 4,6-Diaminoresorcins aus dem Hydrier-Autoklaven, etwa mittels Stickstoff über ein Steigrohr, das mit einer Sintermetall-Fritte versehen ist, zu gewährleisten. Bevorzugt wird Methanol als flüssiges Reaktionsmedium eingesetzt. Das gesamte flüssige Reaktionsmedium für die Hydrierung wird in einer Menge von 2 bis 50 Gew.-Teilen, bevorzugt 5 bis 25 Gew.-Teilen, bezogen auf 1 Gew.-Teil 1,3-Dibenzyloxy-4,6-dinitrobenzol, eingesetzt.

Im einzelnen wird das erfindungsgemäße Verfahren wie folgt durchgeführt: In eine Vorlage aus Schwefelsäure-Monohydrat werden bei 20°C in ca. 1 h unter Kühlung simultan 1,3-Dichlorbenzol und Mischsäure (äquimolares Gemisch von HNO₃ und SO₃ als Oleum 65) eindosiert, wobei das Molverhältnis HNO₃ zu 1,3-Dichlorbenzol beispielsweise etwa 2,2:1 betragen soll. Das Nitriergemisch wird ca. 4 h bei 20°C nachgerührt, bis zu einer maximalen Temperatur von 20°C auf ein Eis/Wasser-Gemisch ausgetragen und die resultierende wässrige Suspension 1 h bei 20°C nachgerührt. Das sehr gut zu filtrierende Produkt wird isoliert, mit Wasser säurefrei gewaschen und gegebenenfalls getrocknet (technisches 4,6/2,4-Dinitro- 1,3-dichlorbenzol-Gemisch).

In eine frisch bereitete Lösung von Na-Benzylat in Benzylalkohol wird bei etwa 10°C unter Stickstoff das erhaltene technische 4,6/2,4-Dinitro-1,3-dichlorbenzol-Gemisch (90:10) in ca. 1/2 h unter Kühlung eingetragen, wobei das Molverhältnis 4,6/2,4-Dinitro-1,3-dichlorbenzol-Gemisch zu Na-Benzylat beispielsweise etwa 1:2,4 betragen soll. Die resultierende Suspension von hauptsächlich 1-Benzyloxy-3-chlor-4,6/2,4-dinitrobenzol-Gemisch wird ca. 1/2 h bei 10°C nachgerührt, auf 30°C erwärmt und bis zur vollständigen Überführung in das Gemisch der Dibenzyloxy-Verbindungen ca. 3 h bei 30°C nachgerührt. Das im Gemisch mit NaCl ausgefallende, sehr gut zu filtrierende 1,3-Dibenzyloxy-4,6-dinitrobenzol wird bei 30°C über eine Glassinternutsche isoliert und gut abgepresst. Zur Abtrennung von NaCl und anhaftendem Benzylalkohol wird das Produkt in der ca. fünffachen Menge Wasser angeschlämmt, 1 h bei Raumtemperatur (20°C) gerührt, erneut filtriert und mit Wasser gewaschen.

In einem Hydrier-Autoklaven wird eine Suspension von Pd(5 %)/A-Kohle-Kontakt in Methanol vorgelegt. Unter Hydrierbedingungen (60°C; 20 bar H₂) wird im Verlaufe von 30 - 90 min. eine 5 - 10 Gew.-%ige Suspension von 1,3-Dibenzyloxy-4,6-dinitrobenzol in Methanol eingepumpt, wobei die Konzentration von 1,3-Dibenzyloxy-4,6-dinitrobenzol, bezogen auf die Gesamtmenge Methanol 5 bis 7,5 Gew.-% und das Gewichtsverhältnis 1,3-Dibenzyloxy-4,6-dinitrobenzol zu Palladium ca. 85:1 betragen soll. Nach beendeter Wasserstoff-Aufnahme wird die Reaktionsmischung ca. 60 min bei 60°C und 20 bar H₂ nachgerührt, der Rührer stillgesetzt und ca. 75 % der methanolischen 4,6-Diaminoresorcin-Lösung aus dem Hydrier-Autoklaven, beispielsweise mittels Stickstoff über ein Steigrohr, das mit einer Sintermetall-Fritte versehen ist, in eine Vorlage auf wässrige Salzsäure abgedrückt. Nach Abdestillieren des organischen Lösungsmittels kann das 4,6-Diaminoresorcin durch Eindampfen der resultierenden salzsauren, wäßrigen Lösung als stabiles Dihydrochlorid isoliert und gegebenenfalls durch Umlösung gereinigt werden. Auf die gleiche Weise können mit dem eingesetzten Pd(5 %)/A-Kohle-Kontakt zahlreiche Hydrierungen nacheinander ohne Abnahme der Katalysator-Aktivität durchgeführt werden, was zu einem sehr niedrigen Katalysator-Verbrauch führt.

Es ist als ausgesprochen überraschend anzusehen, daß es unter den erfindungsgemäßen Bedingungen in Schritt a) gelingt, 1,3-Dichlor-4,6-dinitrobenzol in einer sicherheitstechnisch unbedenklichen Weise in verbesserter Ausbeute und Qualität herzustellen (siehe Vergleichsbeispiele). Bei Verwendung der erfindungsgemäßen Mischsäure kann die Dinitrierung von 1,3-Dichlorbenzol bei wesentlich niedrigeren Temperaturen im Vergleich zum Stand der Technik durchgeführt werden, womit die Bildung höher nitrierter, schlagempfindlicher Produkte vermieden wird. Außerdem kann wegen des wesentlich geringeren Gehaltes an dem isomeren 1,3-Dichlor-2,4-dinitrobenzol auf eine Ausbeute-mindernde Reinigung des Produktes durch Umlösen aus Ethanol verzichtet werden.

Es ist weiterhin als überraschend anzusehen, daß nach dem erfindungsgemäßen Verfahren in Schritt b) bei der bevorzugten Ausführungsform mit Benzylalkohol zugleich als Reaktionspartner und Lösungsmittel 1,3-Dibenzyloxy-4,6-dinitrobenzol selbst dann in reiner Form erhalten wird, wenn 1,3-Dichlor-4,6/2,4-dinitrobenzol-Isomerengemische Isomerengemische in die Reaktion eingesetzt werden, die bis zu 60 % 1,3-Dichlor-2,4-dinitrobenzol, bezogen auf das Gesamtgewicht des Gemisches, enthalten. Das in einem solchen Fall als Hauptprodukt vorliegende isomere 1,3-Dibenzyloxy-2,4-dinitrobenzol bleibt vollständig in Lösung. Außerdem wird nach dem erfindungsgemäßen Verfahren durch die Temperatur-gestufte Reaktion im Vergleich zum Stand der Technik wesentlich weniger Benzaldehyd aus Benzylalkohol in einer unbekannten Nebenreaktion gebildet.

Für Schritt c) des erfindungsgemäßen Verfahrens ist es als überraschend anzusehen, daß mittels katalytischer Pumphydrierung zahlreiche Hydrierungen nacheinander ohne Abnahme der Katalysator-Aktivität durchgeführt werden können, obwohl bekannt ist, daß die entstehenden Amine, wie Amine allgemein, Platinmetall-Katalysatoren im Verlaufe der Reaktion inhibieren, so daß ein Zusatz von Säure zur Bindung der entstehenden Amine empfohlen wird. (Houben-Weyl, Handbuch der organischen Chemie, Band 4; 1 c. S. 509 (1980)).

Das erfindungsgemäße Verfahren kann diskontinuierlich und kontinuierlich durchgeführt werden.

### Beispiele

### Beispiel 1 (Technisches 1,3-Dichlor-4,6/2,4-dinitrobenzol-Gemisch)

In einer Mehrhalskolben-Rührapparatur mit Innenthermometer und zwei Dosiertropftrichtern für 1,3-Dichlorbenzol und Mischsäure wurden 700 g Schwefelsäure-Monohydrat (7,14 Mol) vorgelegt. Unter Rühren und Eiskühlung wurden simultan im Verlaufe von 1 h bei 20°C 147 g 1,3-Dichorbenzol 99,4 %ig (0,99 Mol) und 506 g Mischsäure (2,20 Mol HNO₃) eindosiert, wobei nach ca. 30 Min. die Kristallisation des Nitrierproduktes einsetzte. Die Reaktionsmischung wurde 4 h bei 20°C nachgerührt. In eine Vorlage aus 3 640 g Eis/Wasser-Gemisch wurde die Reaktionsmischung unter Rühren bis zu einer maximalen Temperatur von 20°C im Verlaufe von ca. 30 min. eingetragen. Die resultierende Produkt-Suspension wurde 1 h bei 20°C nachgerührt, das sehr gut zu filtrierende Reaktionsprodukt über eine Glassinternutsche isoliert und mit insgesamt ca. 3 750 g Wasser säurefrei gewaschen und bei 40°C im Vakuum getrocknet.

Es wurden 226 g 1,3-Dichlor-4,6-dinitrobenzol (trocken) erhalten.

Der durch GC bestimmte Gehalt des isoliertes Produktes betrug:
89,7 Gew.-% 1,3-Dichlor-4,6-dinitrobenzol,
10,1 Gew.-% 1,3-Dichlor-2,4-dinitrobenzol,
0,1 Gew.-% 1,3-Dichlor-2-nitrobenzol,
0,1 Gew.-% 1,3-Dichlor4-nitrobenzol.

Die Ausbeute an 1,3-Dichlor-4,6-dinitrobenzol in Form des 4,6/2,4-Dinitro-Gemisches betrug 86,4 % der theoretischen Ausbeute, bezogen auf eingesetztes 1,3-Dichlorbenzol.

Die Mischsäure wurde wie folgt hergestellt: 141,4 g = 2,20 Mol HNO₃ 90 %ig wurden vorgelegt. Unter Eiskühlung und Rühren wurden bis zu einer maximalen Temperatur von 35°C 365 g Oleum 65 (3,0 Mol SO₃; 1,3 Mol H₂SO₄) im Verlaufe von mehreren Stunden eingetropft.

### Beispiel 2 (Technisches 1,3-Dichlor-4,6/2,4-dinitrobenzol-Gemisch)

Eine wie in Beispiel 1 durchgeführte Reaktion, bei der jedoch 2,3 Mol HNO₃ in Form der in Beispiel 1 verwendeten Mischsäure simultan mit 1,0 Mol 1,3-Dichlorbenzol bei 10°C im Verlaufe von 1,5 h in 600 g Schwefelsäure-Monohydrat eindosiert wurde, ergab 228 g 1,3-Dichlor-4,6-dinitrobenzol (trocken). Der durch GC bestimmte Gehalt des isolierten Produktes betrug:
89,8 Gew.-% 1,3-Dichlor-4,6-dinitrobenzol,
9,3 Gew.-% l,3-Dichlor-2,4-dinitrobenzol,
0,2 Gew.-% 1,3-Dichlor-2-nitrobenzol,
0,7 Gew.-% 1,3-Dichlor-4-nitrobenzol.

Die Ausbeute an 1,3-Dichlor-4,6-dinitrobenzol in Form des 4,6/2,4-Dinitro-Gemisches betrug 86,4 % der theoretischen Ausbeute, bezogen auf eingesetztes 1,3-Dichlorbenzol.

### Beispiel 3 (Technisches 1,3-Dichlor-4,6/2,4-dinitrobenzol-Gemisch)

Eine wie in Beispiel 1 durchgeführte Reaktion, bei der jedoch 2,2 Mol HNO₃ in Form der in Beispiel 1 verwendeten Mischsäure simultan mit 1,0 Mol 1,3-Dichlorbenzol bei 40°C im Verlaufe von 2 h in 700 g Schwefelsäure-Monohydrat eindosiert wurde, ergab 220 g 1,3-Dichlor-4,6-dinitrobenzol (trocken). Der durch GC bestimmte Gehalt des isolierten Produktes betrug:
87,6 Gew.-% 1,3-Dichlor-4,6-dinitrobenzol,
11,5 Gew.-% 1,3-Dichlor-2,4-dinitrobenzol,
0,2 Gew.-% 1,3-Dichlor-2-nitrobenzol,
0,7 Gew.-% 1,3-Dichlor-4-nitrobenzol.

Die Ausbeute an 1,3-Dichlor-4,6-dinitrobenzol in Form des 4,6/2,4-Dinitro-Gemisches betrug 81,3 % der theoretischen Ausbeute, bezogen auf eingesetztes 1,3-Dichlorbenzol.

### Beispiel 4

### (Technisches 1,3-Dichlor-4,6/2,4-dinitrobenzol-Gemisch; Vergleichsbeispiel, nicht erfindungsgemäß)

In einer wie in Beispiel 1 beschriebenen Apparatur wurden 795 g Schwefelsäure-Monohydrat (8,1 Mol) vorgelegt. Unter Rühren wurden in ca. 45 min 259 g 1,3-Dichlorbenzol 99,4 %ig (1,75 Mol) eingetropft. Dann wurden 740 g nicht erfindungsgemäße Mischsäure (67 % H₂SO₄, 33 % HNO₃, insgesamt 3,88 Mol HNO₃) im Verlaufe von ca. 4 h eindosiert, wobei nach ca. 40 min. 55°C erreicht waren. Die Temperatur wurde im weiteren Verlaufe gehalten. Die Reaktionsmischung wurde 4 h bei 55°C nachgerührt. In eine Vorlage aus 4 000 g Eis/Wasser-Gemisch wurde die Reaktionsmischung unter Rühren bis zu einer maximalen Temperatur von 20°C im Verlaufe von ca. 30 min. eingetragen. Die resultierende Produkt-Suspension wurde 1 h bei 20°C nachgerührt, das Reaktionsprodukt über eine Glassinternutsche isoliert, mit Wasser säurefrei gewaschen und bei 40°C im Vakuum getrocknet.

Es wurden 374 g 1,3-Dichlor-4,6-dinitrobenzol (trocken) erhalten.

Der durch GC bestimmte Gehalt des isolierten Produktes betrug:
85,4 Gew.-% 1,3-Dichlor-4,6-dinitrobenzol,
14,3 Gew.-% 1,3-Dichlor-2,4-dinitrobenzol,
0,1 Gew.-% 1,3-Dichlor-2-nitrobenzol,
0,2 Gew.-% 1,3-Dichlor-4-nitrobenzol.

Die Ausbeute an 1,3-Dichlor-4,6-dinitrobenzol in Form des 4,6/2,4-Dinitro-Gemisches betrug 76,6 % der theoretischen Ausbeute, bezogen auf eingesetztes 1,3-Dichlorbenzol.

### Beispiel 5

### (Technisches 1,3-Dichlor-4,6/2,4-dinitrobenzol-Gemisch; Vergleichsbeispiel, nicht erfindungsgemäß)

In einer wie in Beispiel 1 beschriebenen Apparatur wurden 336 g nicht erfindungsgemäße Mischsäure (67 % H₂SO₄ 33 % HNO₃; insgesamt 1,76 Mol HNO₃) vorgelegt. Unter Rühren und Kühlung wurden in 1 h bei 5 bis 15°C 64 g 1,3-Dichlorbenzol 99,4 %ig (0,44 Mol) eingetropft. Nach Entfernung des Kühlbades führte die exotherme Reaktion zu einem starken Temperaturanstieg. Die Reaktionsmischung wurde 2 h bei 80°C nachgerührt. In eine Vorlage von 1 800 g Eis/Wasser-Gemisch wurde die Reaktionsmischung unter Rühren bis zu einer maximalen Temperatur von 20°C im Verlaufe von ca. 30 min. eingetragen. Die resultierende Produkt-Suspension wurde 1 h bei 20°C nachgerührt, das Reaktionsprodukt über eine Glassinternutsche isoliert, mit Wasser säurefrei gewaschen und bei 40°C im Vakuum getrocknet.

Es wurden 91 g 1,3-Dichlor-4,6-dinitrobenzol (trocken) erhalten.

Der durch GC bestimmte Gehalt des isolierten Produktes betrug:
84,0 Gew.-% 1,3-Dichlor-4,6-dinitrobenzol,
14,3 Gew.-% 1,3-Dichlor-2,4-dinitrobenzol,
1,7 Gew.-% unbekannte Verbindungen.

Die Ausbeute an 1,3-Dichlor-4,6-dinitrobenzol in Form des 4,6/2,4-Dinitro-Gemisches betrug 73,2 % der theoretischen Ausbeute, bezogen auf eingesetztes 1,3-Dichlorbenzol.

### Beispiel 6 (1,3-Dibenzyloxy-4,6-dinitrobenzol)

In einer Mehrhalskolben-Rührapparatur mit Innenthermometer und Feststoff-Eintrag wurden 1 560 g Benzylalkohol vorgelegt. Unter Durchleiten von trockenem Stickstoff und Eis-Kühlung wurden im Verlauf von ca. 15 min bis zu einer maximalen Temperatur von 25°C 74 g gemahlenes NaOH (1,85 Mol) eingetragen. Die Reaktionsmischung wurde bis zur vollständigen Lösung ca. 12 h bei Raumtemperatur (20°C) nachgerührt. In die gelblich gefärbte, schwach getrübte Reaktionslösung wurden unter Durchleiten von trockenem Stickstoff und Eis/NaCl-Kühlung im Verlaufe von ca. 30 min bei 10°C 183 g 1,3-Dichlor-4,6/2,4-dinitrobenzol-Isomerengemisch (0,685 Mol 1,3-Dichlor-4,6-dinitrobenzol, 0,085 Mol 1,3-Dichlor-2,4-dinitrobenzol) eingetragen. Die resultierende hellgelbe Suspension wurde 30 min bei 10°C nachgerührt, dann auf 30°C erwärmt und ca. 3 h bei dieser Temperatur nachgerührt. Das ausgefallene, sehr gut zu filtrierende Produkt-Gemisch aus 1,3-Dibenzyloxy-4,6-dinitrobenzol und NaCl wurde bei 30°C über eine Glassinternutsche isoliert und zur Entfernung der Benzylalkohol-Mutterlauge gut trocken gesaugt. In eine Vorlage aus 2 500 g Wasser wurde unter Rühren das isolierte Rohprodukt bei 20°C eingetragen, die resultierende Anschlämmung bis zur vollständigen Lösung von NaCl ca. 1 h bei 20°C gerührt, das gereinigte, praktisch farblose Produkt erneut filtriert, bis zur vollständigen Entfernung von Benzylalkohol mit ca. 5 000 g Wasser in mehreren Portionen gewaschen und im Vakuum bei 60°C getrocknet.

Es wurden 237 g Reinprodukt (trocken) erhalten.

Der durch High Pressure Liquid Chromatography (HPLC) bestimmte Gehalt des isolierten Produktes betrug:

| | |
|---|---|
| Gehalt (Titan-Reduktion), MG 380 | 99,8 Gew.-%, davon (HPLC), |
| 1,3-Dibenzyloxy-4,6-dinitrobenzol | 99,1 Gew.-%, |
| 1-Benzyloxy-3-chlor-4,6-dinitrobenzol | 0,6 Gew.-%; |
| 1,3-Dibenzyloxy-2,4-dinitrobenzol | < 0,1 Gew.-%, |
| unbekannte Verbindungen | 0,2 Gew.-%. |

Die Ausbeute an 1,3-Dibenzyloxy-4,6-dinitrobenzol betrug 91,0 % der theoretischen Ausbeute, bezogen auf eingesetztes 1,3-Dichlor-4,6-dinitrobenzol in Form des 1,3-Dichlor-4,6/2,4-dinitrobenzol-Gemisches. Der Benzaldehyd-Gehalt in der Benzylalkohol-Mutterlauge betrug nach GC 0,5 %.

### Beispiel 7 (1,3-Dibenzyloxy-4,6-dinitrobenzol)

Eine wie in Beispiel 6 durchgeführte Reaktion, bei der jedoch eine Mischung aus 1 000 ml Benzylalkohol und 500 ml Toluol verwendet wurde, ergab 236 g Reinprodukt (trocken).

Der durch HPLC bestimmte Gehalt des isolierten Produktes betrug:

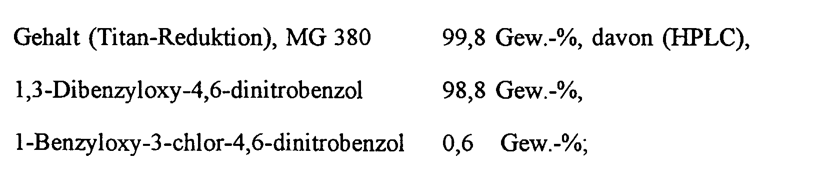

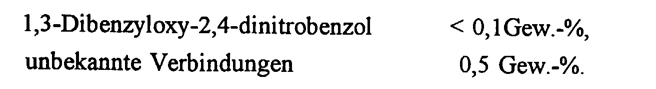

Die Ausbeute an 1,3-Dibenzyloxy-4,6-dinitrobenzol betrug 90,5 % der theoretischen Ausbeute, bezogen auf eingesetztes 1,3-Dichlor-4,6-dinitrobenzol in Form des 1,3-Dichlor-4,6/2,4-dinitrobenzol-Gemisches. Der Benzaldehyd-Gehalt in der Benzylalkohol-Mutterlauge betrug nach GC 0,6 %

### Beispiel 8 (1,3-Dibenzyloxy-4,6-dinitrobenzol)

Eine wie in Beispiel 6 durchgeführte Reaktion, bei der jedoch reines 1,3-Dichlor-4,6-dinitrobenzol (0,77 Mol) verwendet wurde, ergab 271 g Reinprodukt (trocken).

Der durch HPLC bestimmte Gehalt des isolierten Produktes betrug:

| | |
|---|---|
| Gehalt (Titan-Reduktion), MG 380 | 99,9 Gew.-%, davon (HPLC), |
| 1,3-Dibenzyloxy-4,6-dinitrobenzol | 99,1 Gew.-%, |
| 1-Benzyloxy-3-chlor-4,6-dinitrobenzol | 0,8 Gew.-%; |
| unbekannte Verbindungen | 0,1 Gew.-%. |

Die Ausbeute an 1,3-Dibenzyloxy-4,6-dinitrobenzol betrug 92,5 % der theoretischen Ausbeute, bezogen auf eingesetztes 1,3-Dichlor-4,6-dinitrobenzol. Der Benzaldehyd-Gehalt in der Benzylalkohol-Mutterlauge betrug nach GC 0,3 %.

### Beispiel 9 (1,3-Dibenzyloxy-4,6-dinitrobenzol)

Eine wie in Beispiel 6 durchgeführte Reaktion, bei der jedoch ein 1,3-Dichlor-4,6/2,4-dinitrobenzol-Gemisch(0,77 Mol) mit 59 % 1,3-Dichlor-2,4-dinitrobenzol verwendet wurde, ergab 99 g Reinprodukt (trocken).

Der durch HPLC bestimmte Gehalt des isolierten Produktes betrug:

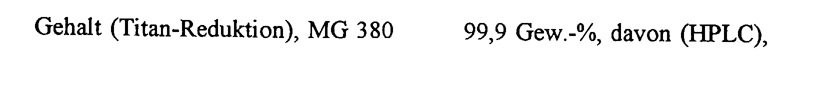

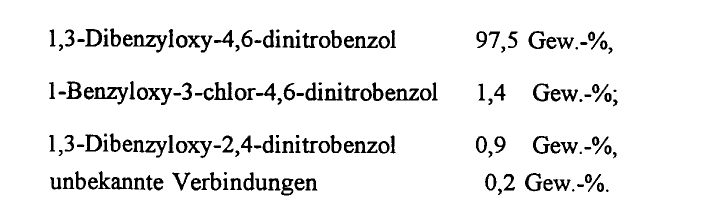

Die Ausbeute an 1,3-Dibenzyloxy-4,6-dinitrobenzol betrug 82,0 % der theoretischen Ausbeute, bezogen auf eingesetztes 1,3-Dichlor-4,6-dinitrobenzol. Der Benzaldehyd-Gehalt in der Benzylalkohol-Mutterlauge betrug nach GC 0,7 %.

### Beispiel 10

### (1,3-Dibenzyloxy-4,6-dinitrobenzol; Vergleichsbeispiel, nicht erfindungsgemäß)

Eine wie in Beispiel 6 durchgeführte Reaktion, bei der jedoch reines 1,3-Dichlor-4,6-dinitrobenzol(0,77 Mol) bis zu einer Temperatur von 50°C in die Na-Benzylat/Benzylalkohol-Lösung eingetragen wurde und die Reaktionsmischung 30 min bei 50°C nachgerührt wurde, ergab 262 g bräunlich gefärbtes Produkt (trocken).

Der durch HPLC bestimmte Gehalt des isolierten Produktes betrug:

| | |
|---|---|
| Gehalt (Titan-Reduktion), MG 380 | 98,9 Gew.-%, davon (HPLC), |
| 1,3-Dibenzyloxy-4,6-dinitrobenzol | 97,8 Gew.-%, |
| 1-Benzyloxy-3-chlor-4,6-dinitrobenzol | 0,4 Gew.-%; |
| unbekannte Verbindungen | 1,8 Gew.-%. |

Die Ausbeute an 1,3-Dibenzyloxy-4,6-dinitrobenzol betrug 88,5 % der theoretischen Ausbeute, bezogen auf eingesetztes 1,3-Dichlor-4,6-dinitrobenzol. Der Benzaldehyd-Gehalt in der Benzylalkohol-Mutterlauge betrug nach GC 2,5 %.

### Beispiel 11 (4,5-Diaminoresorcin)

In einem 1,3 1-Hydrierautoklaven wurde eine Suspension von 15 g Pd (5 %)/A-Kohle-Kontakt (feucht; 6 g trocken; 0,3 g Pd) in 160 g Methanol vorgelegt. Unter Hydrierbedingungen wurden bei 60°C und 20 bar H₂ im Verlaufe von ca. 30 min. 480 g einer 5 gew.-%igen 1,3-Dibenzyloxy-4,6-dinitrobenzol/-Methanol-Suspension (0,063 Mol) eingepumpt. Die Reaktionsmischung wurde ca. 3 h bis zur beendeten Wasserstoff-Aufnahme bei 60°C und 20 bar H₂ nachgerührt, der Rührer stillgesetzt und ca. 75 % der methanolischen 4,6-Diaminoresorcin-Lösung aus dem Hydrierautoklaven mittels Stickstoff über ein Steigrohr, das mit einer Sintermetall-Fritte versehen war, in eine Vorlage von 120 g 10 gew.-%ige wässrige Salzsäure abgedrückt. Nach Abdestillieren des organischen Lösungsmittels wurde das gebildete 4,6-Diaminoresorcin durch Eindampfen der resultierenden salzsauren, wäßrigen Lösung als stabiles Dihydrochlorid isoliert. In die im Hydrierautoklaven verbliebene Suspension des Pd(5 %)/A-Kohle-Kontaktes in ca. 160 g 4,6-Diaminoresorcin/Methanol-Lösung wurden auf die gleiche Weise weitere 480 g der 5 gew.-%igen 1,3-Dibenzyloxy-4,.6-dinitrobenzol/Methanol-Suspension bei 60°C und 20 bar H₂ im Verlaufe von ca. 30 min. eingepumpt und ohne Abnahme der Katalysator-Aktivität hydriert. Auf diese Weise wurden insgesamt fünf Hydrierungen mit viermaliger Wiederbenutzung des Kontaktes nacheinander durchgeführt.

Es wurden insgesamt 64,1g 4,6-Diaminoresorcin-dihydrochlorid (trocken) erhalten.

Nach HPLC war das eingesetzte 1,3-Dibenzyloxy-4,6-dinitrobenzol vollständig umgesetzt und das isolierte 4,6-Diaminoresorcin-dihydrochlorid chromatographisch praktisch einheitlich. Nach ¹HNMR enthielten die isolierten Produkte keine Benzyl-Gruppen.

| | |
|---|---|
| Gehalt (aus Chlor) MG 213 | 97,3 Gew.-%, |
| Gehalt (aus Stickstoff) MG 213 | 98,1 Gew.-%. |

Die Ausbeute an 4,6-Diaminoresorcin betrug 93,0 % der theroetischen Ausbeute (aus Chlor), bezogen auf eingesetztes 1,3-Dibenzyloxy-4,6-dinitrobenzol bzw. 93,7 % der theroretischen Ausbeute (aus Stickstoff), bezogen auf eingesetztes 1,3-Dibenzyloxy-4,6-dinitrobenzol.

### Beispiel 12 (4,6-Diaminoresorcin)

Eine wie in Beispiel 11 durchgeführte Reaktion, bei der jedoch 480 g 7,5 gew.-%ige 1,3-Dibenzyloxy-4,6-dinitrobenzol/Methanol-Suspension (0,095 Mol) eingepumpt wurde, ergab nach insgesamt fünf Hydrierungen mit viermaliger Wiederbenutzung 85,5 g 4,6-Diaminoresorcin-dihydrochlorid (trocken).

| | |
|---|---|
| Gehalt (aus Chlor) MG 213 | 97,0 Gew.-%. |

Die Ausbeute an 4,6-Diaminoresorcin betrug 82,0 % der theroetischen Ausbeute (aus Chlor), bezogen auf eingesetztes 1,3-Dibenzyloxy-4,6-dinitrobenzol.

### Beispiel 13 (4,6-Diaminoresorcin)

Eine wie in Beispiel 11 durchgeführte Reaktion, bei der jedoch im 1,3 l-Hydrierautoklaven eine Suspension von 24 g Pd(5 %)/A-Kohle-Kontakt (feucht; 8,9 g trocken; 0,44 g Pd) in 160 g Methanol vorgelegt wurde, ergab nach insgesamt fünf Hydrierungen mit viermaliger Wiederbenutzung des Kontaktes 63,8 g 4,6-Diaminoresorcin-dihydrochlorid(trocken).

| | |
|---|---|
| Gehalt (aus Chlor) MG 213 | 98,2 Gew.-%. |

Die Ausbeute an 4,6-Diaminoresorcin betrug 93,4 % der theroetischen Ausbeute (aus Chlor), bezogen auf eingesetztes 1,3-Dibenzyloxy-4,6-dinitrobenzol.

## Patentansprüche

1. Verfahren zur Herstellung von 4,6-Diaminoresorcin aus 1,3-Dichlorbenzol durch Dinitrierung zum 1,3-Dichlor-4,6-dinitrobenzol, durch nachfolgende Umsetzung mit Na-Benzylat in Benzylalkohol zum 1,3-Dibenzyloxy-4,6-dinitrobenzol und durch nachfolgende katalytische Hydrierung von 1,3-Di-benzyloxy-4,6-dinitrobenzol an einem Edelmetall-Kontakt, dadurch gekennzeichnet, daß man
a) in einem ersten Schritt 1,3-Dichlorbenzol in wasserfreier Schwefelsäure, die 0 bis 10 Gew.-% freies SO₃ enthält, mit einer Salpetersäure, Schwefelsäure und 0,7 bis 1,5 Mol, bevorzugt 0,8 bis 1,2 Mol, besonders bevorzugt 0,9 bis 1,1 Mol SO₃ pro Mol Salpetersäure enthaltenden Mischsäure in einem Temperaturbereich von 0 bis 40°C umsetzt, wobei das Molverhältnis HNO₃ zu 1,3-Dichlorbenzol 2 bis 3 beträgt,
b) in einem zweiten Schritt die in a) erhaltenen 1,3-Dichlor-4,6/2,4-dinitrobenzol-Isomerengemische, die 0,1 bis 60 % 1,3-Dichlor-2,4-dinitrobenzol, bezogen auf das Gesamtgewicht des Isomerengemisches enthalten, mit 2 bis 5 Mol Benzylalkohol und 2 bis 5 Äquivalenten einer starken Base in Gegenwart eines inerten Lösungsmittels umsetzt, wobei man hierzu stufenweise zunächst im Temperaturbereich vom -15 bis +15°C und dann im Temperaturbereich von 20-40°C arbeitet und
c) in einem dritten Schritt das in b) erhaltene 1,3-Dibenzyloxy-4,6-dinitrobenzol an einem Edelmetall-Kontakt in einem inerten organischen Lösungsmittel bei einem H₂-Druck von 1 bis 100 bar und einer Temperatur von 20 bis 100°C durch eine katalytische Pumphydrierung zum 4,6-Diaminoresorcin umsetzt.

2. Verfahren gemäß Anspruch 1, dadurch gekennzeichnet, daß pro Mol 1,3-Dichlorbenzol 2,1 bis 2,5 Mol Salpetersäure, bevorzugt 2,15 bis 2,30 Mol Salpetersäure, in Form der Mischsäure eingesetzt werden.

3. Verfahren gemäß Anspruch 1, dadurch gekennzeichnet, daß in eine Vorlage aus wasserfreier Schwefelsäure simultan 1,3-Dichlorbenzol und Mischsäure eindosiert werden.

4. Verfahren gemäß Anspruch 1, dadurch gekennzeichnet, daß man als starke Basen eine oder mehrere aus der Gruppe der Alkalimetalle, Erdalkalimetalle, Alkalimetallhydroxide, Erdalkalimetallhydroxide, Alkalimetallcarbonate und Erdalkalimetallcarbonate, bevorzugt aus der Gruppe der Alkalimetalle, Alkalimetallhydroxide und Alkalimetallcarbonate, besonders bevorzugte aus der Gruppe Natriummetall, Natriumhydroxid und Natriumcarbonat, einsetzt.

5. Verfahren gemäß Anspruch 1, dadurch gekennzeichnet, daß als inertes Lösungsmittel ein aliphatischer, aromatischer oder alkylaromatischer Kohlenwasserstoff, ein halogenierter aromatischer Kohlenwasserstoff oder zusätzlicher Benzylalkohol, bevorzugt zusätzlicher Benzylalkohol, in einer Menge von 3 bis 20 Gew.-Teilen, bevorzugt 5 bis 15 Gew.-Teilen, bezogen auf 1 Gew.-Teil Dichlor-dinitrobenzol, eingesetzt wird.

6. Verfahren gemäß Anspruch 1, dadurch gekennzeichnet, daß eine Temperatur-gestufte Reaktion in der ersten Stufe in einem Temperaturbereich von 5 bis 15°C, und in einer zweiten Reaktionsstufe in einem Temperaturbereich von 25 bis 35°C durchgeführt wird.

7. Verfahren gemäß Anspruch 1, dadurch gekennzeichnet, daß als flüssiges Reaktionsmedium für die katalytische Pumphydrierung ein Alkohol, ein Ether, ein aromatischer oder alkylaromatischer Kohlenwasserstoff, eine organische Säure oder ein Gemisch mehrerer von ihnen in einer Menge von 2 bis 50 Gew.-Teilen, bevorzugt 5 bis 25 Gew.-Teilen, bezogen auf 1 Gew.-Teil 1,3-Dibenzyloxy-4,6-dinitrobenzol, eingesetzt wird.

8. Verfahren gemäß Anspruch 1, dadurch gekennzeichnet, daß als flüssiges Reaktionsmedium für die katalytische Pumphydrierung Methanol, Ethanol, Propanol, Isopropylalkohol, Butanol, Isobutanol, Tetrahydrofuran, Dioxan, Benzol, Toluol, Xylol, Dimethoxy-ethan, Diethoxy-ethan, 1,2-Dimethoxy-propan und andere Glykolmono- und diether, Essigsäure, Propionsäure oder ein Gemisch mehrerer von ihnen eingesetzt wird, wobei im Falle wasserlöslicher Reaktionsmedium bis zu 75 Gew.-% dieses Reaktionsmediums durch Wasser ersetzt sein kann.

9. Verfahren gemäß Anspruch 1, dadurch gekennzeichnet, daß die katalytische Pumphydrierung in Methanol am Palladium/A-Kohle-Kontakt in einer Menge von 0,2 bis 2 Gew.-% des Platinmetalls, bezogen auf das Gewicht von 1,3-Dibenzyloxy-4,6-dinitrobenzol, bei einem H₂-Druck von 10 bis 30 bar und im Temperaturbereich von 40 bis 80°C, durchgeführt wird, wobei Methanol in einer Menge von 5 bis 25 Gew.-Teilen, bezogen auf 1 Gew.-Teil 1,3-Dibenzyloxy-4,6-dinitrobenzol, eingesetzt wird.

## Claims

1. Process for the preparation of 4,6-diaminoresorcinol from 1,3-dichlorobenzene by dinitration to give 1,3-dichloro-4,6-dinitrobenzene, by subsequent reaction with sodium benzylate in benzyl alcohol to give 1,3-dibenzyloxy-4,6-dinitrobenzene and by subsequent catalytic hydrogenation of 1,3-dibenzyloxy-4,6-dinitrobenzene on a noble metal catalyst, characterized in that
a) in a first step, 1,3-dichlorobenzene is reacted with a mixed acid containing nitric acid, sulphuric acid and 0.7 to 1.5 mol, preferably 0.8 to 1.2 mol, particularly preferably 0.9 to 1.1 mol, of SO₃ per mol of nitric acid in a temperature range from 0 to 40°C in anhydrous sulphuric acid which contains 0 to 10 % by weight of free SO₃, the molar ratio of HNO₃ to 1,3-dichlorobenzene being 2 to 3,
b) in a second step, the 1,3-dichloro-4,6/2,4-dinitrobenzene isomeric mixtures obtained in a) which contain 0.1 to 60 % of 1,3-dichloro-2,4-dinitrobenzene, based on the total weight of the isomeric mixture, are reacted with 2 to 5 mol of benzyl alcohol and 2 to 5 equivalents of a strong base in the presence of an inert solvent, the process being carried out stepwise initially in the temperature range from -15 to +15°C and then in the temperature range 20-40°C and
c) in a third step the 1,3-dibenzyloxy-4,6-dinitrobenzene obtained in b) is converted to 4,6-diaminoresorcinol by catalytic pumped hydrogenation on a noble metal catalyst in an inert organic solvent at an H₂ pressure of 1 to 100 bar and a temperature of 20 to 100°C.

2. Process according to Claim 1, characterized in that per mol of 1,3-dichlorobenzene, 2.1 to 2.5 mol of nitric acid, preferably 2.15 to 2.30 mol of nitric acid, are used in the form of the mixed acid.

3. Process according to Claim 1, characterized in that 1,3-dichlorobenzene and mixed acid are added simultaneously to a portion of anhydrous sulphuric acid.

4. Process according to Claim 1, characterized in that the strong bases used are one or more selected from the group consisting of the alkali metals, alkaline earth metals, alkali metal hydroxide, alkaline earth metal hydroxides, alkali metal carbonates and alkaline earth metal carbonates, preferably selected from the group consisting of the alkali metals, alkali metal hydroxides and alkali metal carbonates, particularly preferably selected from the group consisting of sodium metal, sodium hydroxide and sodium carbonate.

5. Process according to Claim 1, characterized in that the inert solvent used is an aliphatic, aromatic or alkylaromatic hydrocarbon, a halogenated aromatic hydrocarbon or additional benzyl alcohol, preferably additional benzyl alcohol, in an amount of 3 to 20 parts by weight, preferably 5 to 15 parts by weight, based on 1 part by weight of dichloro-dinitrobenzene.

6. Process according to Claim 1, characterized in that a temperature-stepped reaction is carried out in the first step in a temperature range from 5 to 15°C and in a second reaction step in a temperature range from 25 to 35°C.

7. Process according to Claim 1, characterized in that the liquid reaction medium used for the catalytic pumped hydrogenation is an alcohol, an ether, an aromatic or alkylaromatic hydrocarbon, an organic acid or a mixture of a plurality thereof in an amount of 2 to 50 parts by weight, preferably 5 to 25 parts by weight, based on 1 part by weight of 1,3-dibenzyloxy-4,6-dinitrobenzene.

8. Process according to Claim 1, characterized in that the liquid reaction medium used for the catalytic pumped hydrogenation is methanol, ethanol, propanol, isopropanol alcohol, butanol, isobutanol, tetrahydrofuran, dioxane, benzene, toluene, xylene, dimethoxy-ethane, diethoxy-ethane, 1,2-dimethoxypropane and other glycol mono- and diethers, acetic acid, propionic acid or a mixture of a plurality thereof, where in the case of a water-soluble reaction medium, this reaction medium can be replaced by water up to the extent of 75 % by weight.

9. Process according to Claim 1, characterized in that the catalytic pumped hydrogenation is carried out in methanol on the palladium/activated carbon catalyst in an amount of 0.2 to 2 % by weight of the platinum group metal, based on the weight of 1,3-dibenzyloxy-4,6-dinitrobenzene, at an H₂ pressure of 10 to 30 bar and in the temperature range from 40 to 80°C, where methanol is used in an amount of 5 to 25 parts by weight, based on 1 part by weight of 1,3-dibenzyloxy-4,6-dinitrobenzene.

## Revendications

1. Procédé de préparation du 4,6-diaminorésorcinol à partir du 1,3-dichlorobenzène par dinitration en 1,3-dichloro-4,6-dinitrobenzène, réaction subséquente avec le benzylate de sodium dans l'alcool benzylique, donnant le 1,3-dibenzyloxy-4,6-dinitrobenzène qu'on soumet à hydrogénation catalytique sur un catalyseur à base de métal noble, caractérisé en ce que :
a) dans un premier stade opératoire, on fait réagir le 1,3-dichlorobenzène dans l'acide sulfurique anhydre contenant de 0 à 10 % en poids de SO₃ libre, avec un mélange sulfonitrique contenant de l'acide nitrique, de l'acide sulfurique et 0,7 à 1,5 mole, de préférence 0,8 à 1,2 mole et plus spécialement 0,9 à 1,1 mole de SO₃ par mole d'acide nitrique, dans l'intervalle de température de 0 à 40°C, à un rapport molaire HNO₃/1,3-dichlorobenzène de 2 à 3 ;
b) dans un deuxième stade, on fait réagir le mélange d'isomères 1,3-dichloro-4,6/2,4-dinitrobenzènes contenant 0,1 à 60% de 1,3-dichloro-2,4-dinitrobenzène par rapport au poids total du mélange d'isomères, avec 2 à 5 moles d'alcool benzylique et 2 à 5 équivalents d'une base forte en présence d'un solvant inerte, en opérant par paliers, d'abord dans l'intervalle de températures de -15 à +15°C, puis dans l'intervalle de températures de 20 à 40°C, et
c) dans un troisième stade, on convertit le 1,3-dibenzyloxy-4,6-dinitrobenzène obtenu en b) en le 4,6-diaminorésorcinol par une hydrogénation catalytique sous pompage sur un catalyseur à base de métal noble, dans un solvant organique inerte, à une pression d'hydrogène de 1 à 100 bars et une température de 20 à 100°C.

2. Procédé selon la revendication 1, caractérisé en ce que, pour une mole de 1,3-dichlorobenzène, on utilise de 2,1 à 2,5 moles d'acide nitrique, de préférence de 2,15 à 2,30 moles d'acide nitrique sous la forme du mélange sulfonitrique.

3. Procédé selon la revendication 1, caractérisé en ce que l'on introduit simultanément le 1,3-dichlorobenzène et le mélange sulfonitrique dans l'acide sulfurique anhydre.

4. Procédé selon la revendication 1, caractérisé en ce que l'on utilise en tant que bases fortes, isolément ou en combinaison entre eux, des métaux alcalins, des métaux alcalino-terreux, des hydroxydes de métaux alcalins, des hydroxydes de métaux alcalino-terreux, des carbonates de métaux alcalins et des carbonates de métaux alcalino-terreux, de préférence des métaux alcalins, des hydroxydes de métaux alcalins et des carbonates de métaux alcalins, et plus spécialement le sodium métallique, l'hydroxyde de sodium ou le carbonate de sodium.

5. Procédé selon la revendication 1, caractérisé en ce que l'on utilise en tant que solvant inerte un hydrocarbure aliphatique, aromatique, alkyl-aromatique, un hydrocarbure aromatique halogéné ou bien un complément d'alcool benzylique, de préférence un complément d'alcool benzylique, en quantité de 3 à 20 parties en poids, de préférence de 5 à 15 parties en poids pour une partie en poids du dichlorodinitrobenzène.

6. Procédé selon la revendication 1, caractérisé en ce que l'on procède à une réaction par paliers en fonction de la température, en observant au premier stade un intervalle de température de 5 à 15°C et au deuxième stade de réaction un intervalle de température de 25 à 35°C.

7. Procédé selon la revendication 1, caractérisé en ce que l'on utilise en tant que milieu de réaction liquide pour l'hydrogénation catalytique sous pompage un alcool, un éther, un hydrocarbure aromatique ou alkylaromatique, un acide organique ou un mélange de plusieurs d'entre eux, en quantité de 2 à 50 parties en poids, de préférence de 5 à 25 parties en poids pour une partie en poids de 1,3-dibenzyloxy-4,6-dinitrobenzène.

8. Procédé selon la revendication 1, caractérisé en ce que l'on utilise en tant que milieu de réaction liquide pour l'hydrogénation catalytique sous pompage le méthanol, l'éthanol, le propanol, l'isopropanol, le butanol, l'isobutanol, le tétrahydrofuranne, le dioxanne, le benzène, le toluène, le xylène, le diméthoxyéthane, le diéthoxyéthane, le 1,2-diméthoxypropane ou d'autres mono- et diéthers de glycols, l'acide acétique, l'acide propionique ou un mélange de plusieurs d'entre eux, ce milieu de réaction pouvant être remplacé en proportions allant jusqu'à 75 % de son poids par l'eau lorsqu'il est lui-même soluble dans l'eau.

9. Procédé selon la revendication 1, caractérisé en ce que l'hydrogénation catalytique par pompage est réalisée dans le méthanol, sur un catalyseur au palladium sur charbon actif en quantité correspondant à 0,2 à 2 % en poids de métal de la série du platine par rapport au poids du 1,3-dibenzyloxy-4,6-dinitrobenzène, sous pression d'hydrogène de 10 à 30 bars et dans l'intervalle de température de 40 à 80°C, le méthanol étant mis en oeuvre en quantité de 5 à 25 parties en poids pour 1 partie en poids du 1,3-dibenzyloxy-4,6-dinitrobenzène.
